# EUROPEAN PATENT APPLICATION

(11) **EP 2 266 456 A1**
(43) Date of publication of application: **29.12.2010**
(21) Application number: 09723564.2
(22) Date of filing: 04.03.2009
(51) Int. Cl.: A61B 5/00, A61B 5/145

(54) **METHOD OF ANALYZING BIOLOGICAL COMPONENT, BIOLOGICAL COMPONENT ANALYZER, REACTION CARTRIDGE OF BIOLOGICAL COMPONENT ANALYZER AND EXTRACTION CARTRIDGE OF BIOLOGICAL COMPONENT ANALYZER**

(30) Priority: 19.03.2008 JP 2008070652
(71) Applicant: Sysmex Corporation, Kobe-shi, Hyogo 651-0073 (JP)
(72) Inventor: ASAKURA, Yoshihiro, Kobe-shi Hyogo 651-0073 (JP)
(74) Representative: HOFFMANN EITLE
(86) International application number: PCT/JP2009/054029
(87) International publication number: WO 2009/116390

(57) **Abstract**

This method of analyzing a biological component includes steps of arranging an extraction medium holding device on the skin of a subject, extracting an extract from the subject, attaching a reaction cartridge configured to be detachable on an extraction medium holding part of the extraction medium holding device and to be capable of transferring an extraction medium to a reaction part to the extraction medium holding part of the extraction medium holding device arranged on the skin, and transferring the extraction medium held in the extraction medium holding part to the reaction part.

## Description

### Technical Field

The present invention relates to a method of analyzing a biological component, a biological component analyzer, a reaction cartridge of a biological component analyzer and an extraction cartridge of a biological component analyzer, and more particularly, it relates to a method of analyzing a biological component, a biological component analyzer, a reaction cartridge of a biological component analyzer and an extraction cartridge of a biological component analyzer measuring a biological component extracted from the organism.

### Background Art

A biological component analyzer measuring a biological component extracted from the organism is known in general. Such a biological component analyzer is disclosed in U.S. Patent Laying-Open No. 2005-169799, for example.

The biological component analyzer disclosed in U.S. Patent Laying-Open No. 2005-169799 comprises an extraction/reaction chamber holding glucose (biological component) extracted from the cutaneous layer of the arm of a subject and allowing the glucose to cause a prescribed reaction, a detector detecting the extracted glucose, a control part analyzing a detection result of the detector and calculating the blood glucose level and a display displaying the blood glucose level calculated by the control part. The subject measures the blood glucose level by mounting this apparatus on his/her arm.

However, the conventional biological component analyzer disclosed in the aforementioned U.S. Patent Laying-Open No. 2005-169799 is provided with a large number of components such as the extraction/reaction chamber, the detector, the control part and the display, and hence there is such inconvenience that the overall apparatus increases in size and the apparatus increases in weight. Thus, there is such a problem that mounting such a large-size biological component analyzer on his/her arm during all periods of extraction, reaction, detection, analysis and display of glucose is a burden to the subject.

### Disclosure of the Invention

The present invention has been proposed in order to solve the aforementioned problems, and an object of the present invention is to provide a method of analyzing a biological component, a biological component analyzer, a reaction cartridge of a biological component analyzer and an extraction cartridge of a biological component analyzer allowing for reduction in a burden on the subject.

In order to attain the aforementioned object, a method of analyzing a biological component according to a first aspect of the present invention comprises steps of arranging an extraction medium holding device comprising an extraction medium holding part configured to be capable of holding an extraction medium for holding an extract extracted from a subject on the skin of the subject, extracting the extract from the subject to the extraction medium held in the extraction medium holding part, attaching a reaction cartridge comprising a reaction part allowing the extract to cause a prescribed reaction, configured to be detachable on the extraction medium holding part of the extraction medium holding device and to be capable of transferring the extraction medium held in the extraction medium holding part to the reaction part to the extraction medium holding part of the extraction medium holding device arranged on the skin, transferring the extraction medium held in the extraction medium holding part to the reaction part, detecting the extract from the prescribed reaction caused in the reaction part, and analyzing a result of detecting the extract.

In the aforementioned method of analyzing a biological component according to the first aspect, the step of transferring the extraction medium to the reaction part preferably includes a step of transferring the extraction medium through a transfer passage provided in the reaction cartridge for transferring the extraction medium to the reaction part.

In the aforementioned method of analyzing a biological component having the step of transferring the extraction medium through the transfer passage, the step of transferring the extraction medium to the reaction part preferably includes a step of transferring the extraction medium held in the extraction medium holding part to the reaction part through the transfer passage by capillarity.

A biological component analyzer according to a second aspect of the present invention comprises an extraction medium holding device having an extraction medium holding part configured to be capable of holding an extraction medium for holding an extract extracted from a subject, a reaction cartridge including a reaction part allowing the extract to cause a prescribed reaction and a transfer passage for transferring the extraction medium held in the extraction medium holding part to the reaction part, configured to be detachable on the extraction medium holding part of the extraction medium holding device, a detecting part detecting the extract from the prescribed reaction caused in the reaction part, and an analysis part analyzing a result of detecting the extract.

In the aforementioned biological component analyzer according to the second aspect, the transfer passage is preferably configured to transfer the extraction medium held in the extraction medium holding part to the reaction part by capillarity.

In the aforementioned biological component analyzer according to the second aspect, the transfer passage is preferably configured to come into contact with the extraction medium held in the extraction medium holding part when the reaction cartridge is attached to the extraction medium holding part of the extraction medium holding device.

The aforementioned biological component analyzer according to the second aspect is preferably provided with a detecting device including the detecting part and a cartridge holding part detachably holding the reaction cartridge.

In this case, the detecting device preferably includes the analysis part.

The aforementioned biological component analyzer according to the second aspect preferably further comprises transfer monitoring means monitoring that the extraction medium has been transferred from the extraction medium holding part to the reaction part.

In the aforementioned biological component analyzer according to the second aspect, the extraction medium holding device preferably includes an extraction cartridge having the extraction medium holding part and a mounting part detachably holding the extraction cartridge, detachably mounted on an extract extraction position of the subject.

In this case, the extraction cartridge preferably includes a liquid tank storing the extraction medium held in the extraction medium holding part, connected to the extraction medium holding part.

The aforementioned biological component analyzer according to the second aspect, the extraction medium holding device preferably further comprises an extraction promoting part promoting extraction of the extract.

The aforementioned biological component analyzer according to the second aspect, the extract is preferably glucose, and the analysis part preferably analyzes a detection result of the detecting part and acquires a blood glucose level.

A reaction cartridge according to a third aspect of the present invention is employed in a biological component analyzer analyzing an extract extracted from a subject and comprises a reaction part allowing the extract to cause a prescribed reaction and a transfer passage configured to transfer an extraction medium to the reaction part by capillarity.

An extraction cartridge according to a fourth aspect of the present invention is employed in a biological component analyzer analyzing an extract extracted from a subject and comprises an extraction medium holding part holding an extraction medium, a liquid tank storing the extraction medium held in the extraction medium holding part, connected to the extraction medium holding part, and an air vent provided in the extraction medium holding part for moving the extraction medium from the liquid tank to the extraction medium holding part.

### Brief Description of the Drawings

[Fig. 1] A perspective view showing a blood glucose meter according to an embodiment of the present invention.
[Fig. 2] A perspective view showing an extraction unit of the blood glucose meter according to the embodiment shown in Fig. 1.
[Fig. 3] A perspective view showing a state where the extraction unit of the blood glucose meter according to the embodiment shown in Fig. 1 is mounted on the arm of a subject.
[Fig. 4] A block diagram for illustrating the detailed structure of the blood glucose meter according to the embodiment shown in Fig. 1.
[Fig. 5] A plane view showing an extraction cartridge attached to the extraction unit of the blood glucose meter according to the embodiment shown in Fig. 1.
[Fig. 6] A sectional view taken along the line 200-200 shown in Fig. 5.
[Fig. 7] A plane view showing a detecting unit of the blood glucose meter according to the embodiment shown in Fig. 1.
[Fig. 8] A plane view for illustrating the structure of the detecting unit of the blood glucose meter according to the embodiment shown in Fig. 1.
[Fig. 9] A plane view for illustrating the structure of a reaction cartridge attached to the detecting unit of the blood glucose meter according to the embodiment shown in Fig. 1.
[Fig. 10] A sectional view taken along the line 300-300 shown in Fig. 9.
[Fig. 11] A sectional view taken along the line 400-400 shown in Fig. 9.
[Fig. 12] A diagram for illustrating absorbance of a sensor part of the reaction cartridge attached to the detecting unit of the blood glucose meter according to the embodiment shown in Fig. 1.
[Fig. 13] A diagram for illustrating a use procedure of the blood glucose meter according to the embodiment shown in Fig. 1.
[Fig. 14] A diagram for illustrating the use procedure of the blood glucose meter according to the embodiment shown in Fig. 1.
[Fig. 15] A diagram for illustrating the use procedure of the blood glucose meter according to the embodiment shown in Fig. 1.
[Fig. 16] A diagram for illustrating the use procedure of the blood glucose meter according to the embodiment shown in Fig. 1.
[Fig. 17] A sectional view showing the skin on which micropores are formed by a micropore forming device shown in Fig. 14.
[Fig. 18] A flow chart for illustrating a procedure when the subject employs the blood glucose meter according to the embodiment shown in Fig. 1.
[Fig. 19] A flow chart for illustrating operations of a control part when extracting glucose in the extraction unit of the blood glucose meter according to the embodiment shown in Fig. 1.
[Fig. 20] A flow chart for illustrating operations of the control part when detecting the glucose and measuring the blood glucose level in the detecting unit of the blood glucose meter according to the embodiment shown in Fig. 1. Best Modes for Carrying Out the Invention

An embodiment of the present invention will be hereinafter described with reference to the drawings.

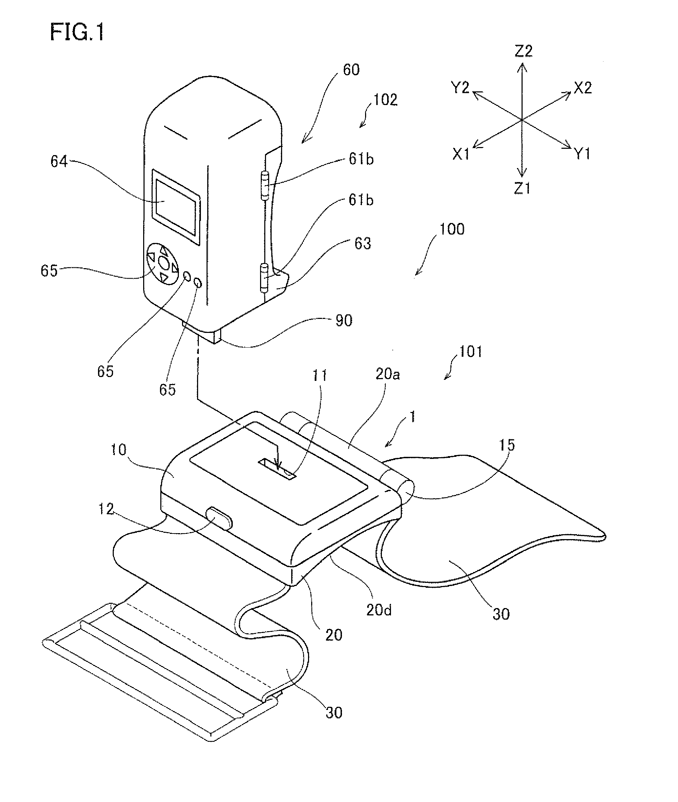

The structure of a blood glucose meter 100 according to the embodiment will be now described with reference to Figs. 1 to 12.

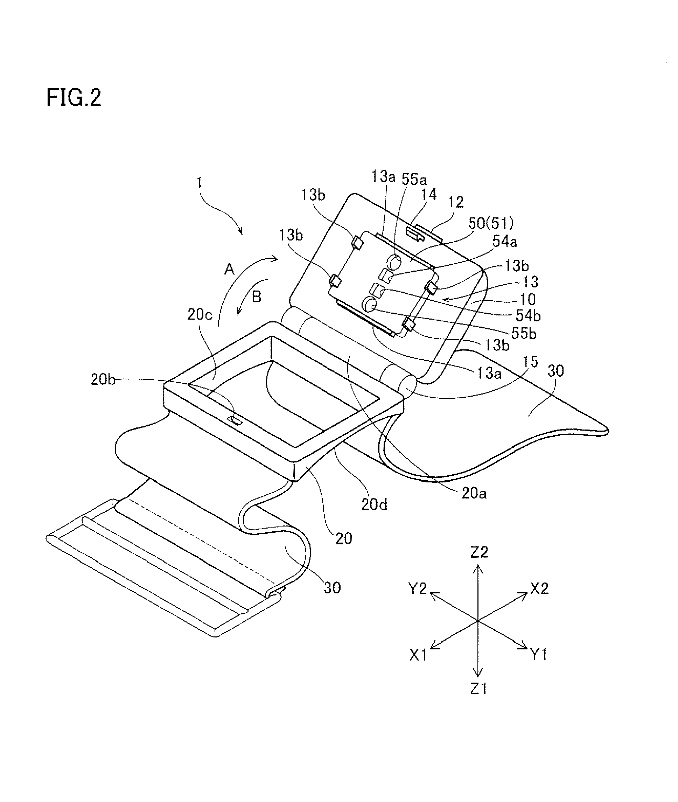

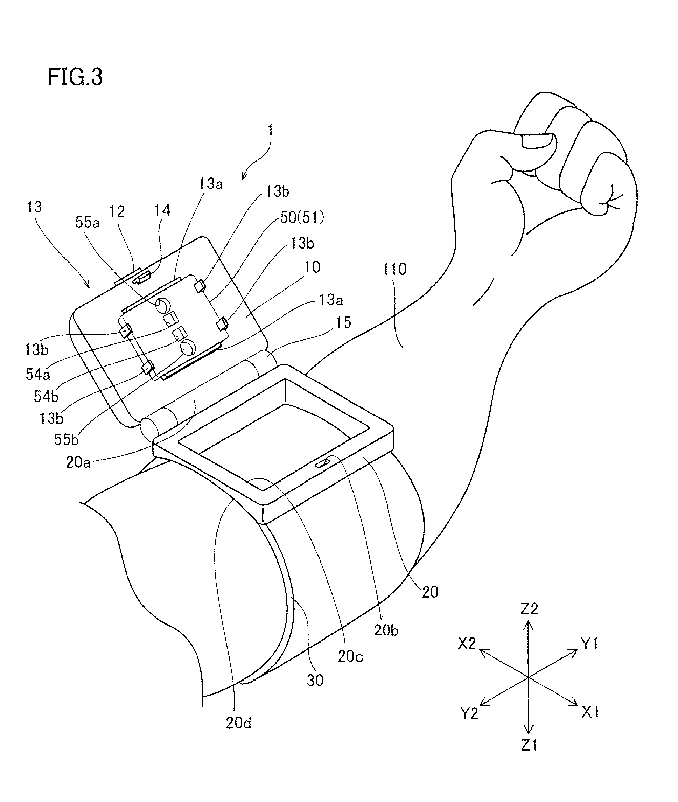

The blood glucose meter 100 according to the embodiment is an apparatus extracting glucose which is one of biochemical components from an organism and calculating a blood glucose level by analyzing the extracted glucose. This blood glucose meter 100 comprises an extraction medium holding device 101 comprising an extraction unit 1 and an extraction cartridge 50 (see Fig. 2) being detachable on the extraction unit 1 and a detecting device 102 comprising a detecting unit 60 and a reaction cartridge 90 being detachable on the detecting unit 60, as shown in Fig. 1. The extraction unit 1 includes a device body 10 detachably holding the extraction cartridge 50, a holding member 20 rotatably holding the device body 10 and a mounting belt 30 for detachably mounting the holding member 20 on the arm 110 (see Fig. 3) of a subject.

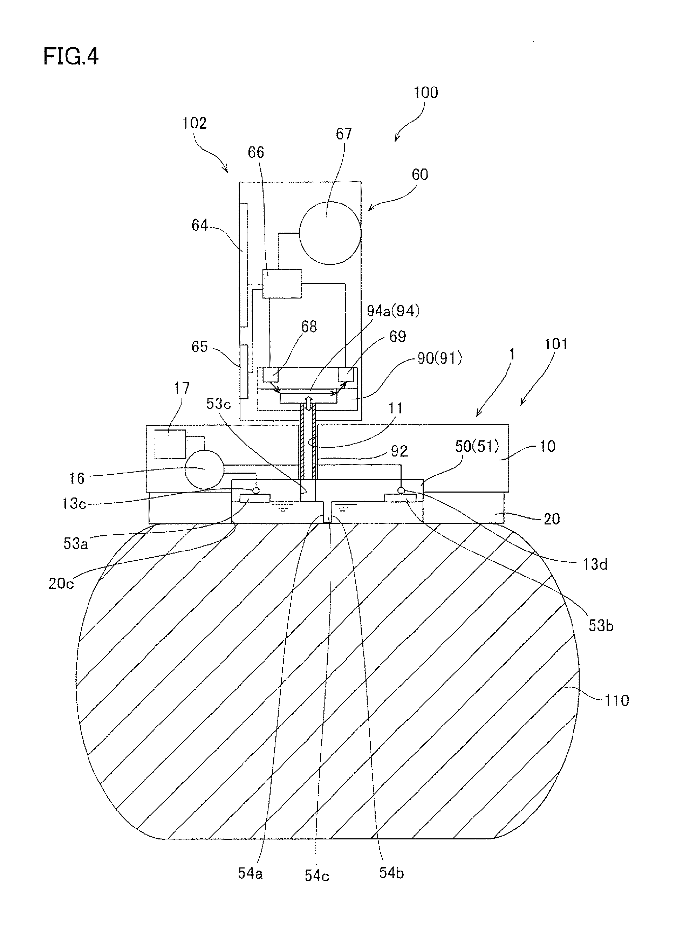

The device body 10 of the extraction unit 1 includes a detecting unit mounting hole 11 arranged on a front surface side (arrow Z2 direction side in Fig. 1), being capable of mounting the reaction cartridge 90 mounted on the detecting unit 60, a release button 12 arranged on an end side in an arrow X1 direction in Fig. 1, an extraction cartridge attachment part 13 (see Fig. 2) and an engaging pawl 14 (see Fig. 2) arranged on a back surface side, a hinge 15 arranged on an end side in an arrow X2 direction in Fig. 1, a direct-current system constant-voltage power supply 16 (see Fig. 4) provided internally, and a control part 17 (see Fig. 4) controlling the constant-voltage power supply 16, as shown in Figs. 1 and 2. The detecting unit mounting hole 11 of the device body 10 is provided to pass through the device body 10 of the extraction unit 1 and is configured to allow a transfer passage 92 of the reaction cartridge 90, described later, mounted on the detecting unit 60 to be inserted therein, as shown in Fig. 4. The release button 12 of the device body 10 is provided for releasing engagement of an engaging hole 20b of the holding member 20 described later and the engaging pawl 14 of the device body 10, as shown in Fig. 2.

The extraction cartridge attachment part 13 of the device body 10 includes a pair of rib-shaped projecting parts 13a functioning as guides when attaching the extraction cartridge 50 and four engaging parts 13b for attaching the extraction cartridge 50, as shown in Figs. 2 and 3.

The four engaging parts 13b of the extraction cartridge attachment part 13 are elastically deformably provided thereby allowing the extraction cartridge 50 to be attached and detached. The engaging pawl 14 of the device body 10 is provided to engage with the engaging hole 20b of the holding member 20 described later when the device body 10 is in a closed state (see Fig. 1) with respect to the holding member 20. The hinge 15 of the device body 10 is configured to be capable of rotating around an axis line extending in an arrow Y1 direction and an arrow Y2 direction.

The constant-voltage power supply 16 is connected to a pair of terminals 13c and 13d provided in the extraction cartridge attachment part 13 of the device body 10, as shown in Fig. 4. The pair of these terminals 13c and 13d are configured to be in contact with electrodes 53a and 53b of the extraction cartridge 50 described later, respectively. The control part 17 has a function of applying a voltage to the electrodes 53a and 53b described later by controlling the constant-voltage power supply 16. In other words, the control part 17 is provided for controlling the constant-voltage power supply 16 to be capable of smoothly extracting glucose from the arm 110 of the subject.

The holding member 20 is configured to rotatably hold the device body 10 and to be capable of being mounted on the arm 110 of the subject with no space therebetween, as shown in Fig. 3. More specifically, the holding member 20 includes a hinge holding part 20a holding the hinge 15 of the device body 10, the engaging hole 20b provided on an end side in the arrow X1 direction in Fig. 3, and an opening 20c provided in the vicinity of a central part in plan view. The holding member 20 is formed of ABS resin or the like. A lower surface part 20d of the holding member 20 is formed in an arc shape when viewed from an arrow Y1 direction. Thus, contact with the arm 110 of the subject can be improved when the holding member 20 is mounted on the arm 110 of the subject by the mounting belt 30.

The device body 10 is attached to a pair of the hinge holding parts 20a of the holding member 20 to be rotatable in directions A and B (see Fig. 1) with respect to the holing member 20 through the hinge 15. The engaging hole 20b of the holding member 20 engages with the engaging pawl 14 of the device body 10 when the device body 10 is in a closed state (see Fig. 1) with respect to the holding member 20 thereby having a function of limiting rotation of the device body 10 in a direction A (opening direction) with respect to the holding member 20. At this time, the extraction cartridge 50 is in contact with a surface of the arm 110 of the subject exposed from the opening 20c of the holding member 20 as shown in Fig. 4, when the extraction cartridge 50 is attached to the device body 10.

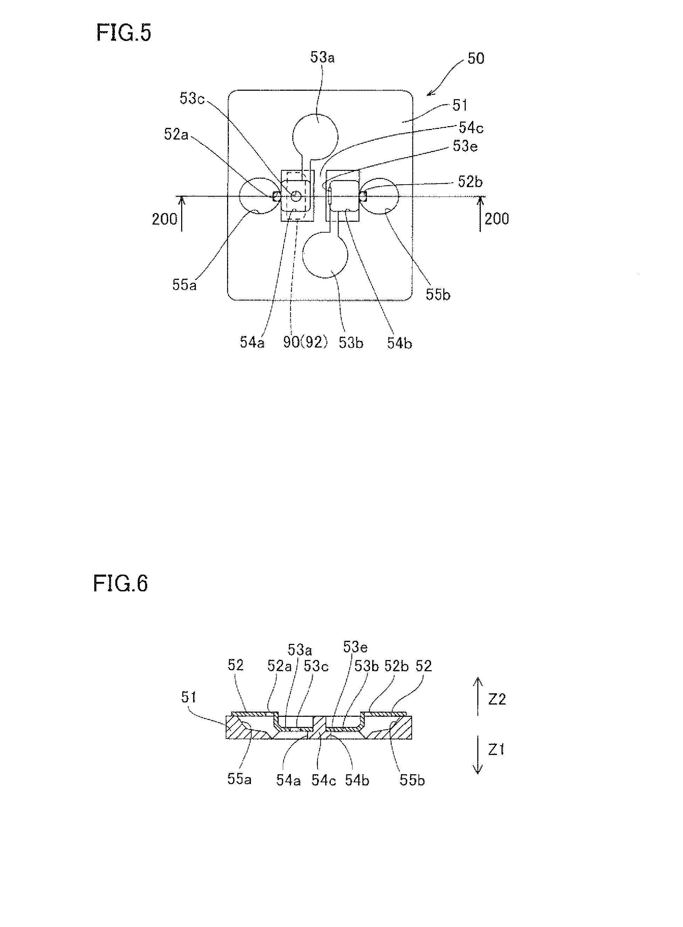

According to the embodiment, the extraction cartridge 50 is configured by a cartridge body 51 made of acrylic resin or the like, a tape 52 (see Fig. 6) attached to a first surface (surface on an arrow Z2 direction side in Fig. 6) of each of a pair of liquid tanks 55a and 55b described later, and the electrodes 53a and 53b connected to a pair of extraction medium holding parts 54a and 54b described later respectively, as shown in Figs. 5 and 6. The cartridge body 51 is integrally provided with the pair of extraction medium holding parts 54a and 54b and the liquid tanks 55a and 55b storing an extraction medium supplied to the pair of extraction medium holding parts 54a and 54b.

The pair of extraction medium holding parts 54a and 54b of the cartridge body 51 are provided on a side (an arrow Z1 direction side in Fig. 6) of a surface coming into contact with a surface of the arm 110 of the subject when the device body 10 is in a closed state (see Fig. 1) with respect to the holding member 20.

Outer peripheral parts of these extraction medium holding parts 54a and 54b are integrally formed with the cartridge body 51, and parts of the extraction medium holding parts 54a and 54b on the arrow Z2 direction (see Fig. 6) side are covered with the electrodes 53a and 53b, as shown in Fig 6. The extraction medium holding parts 54a and 54b are formed separately from each other. More specifically, a dividing part 54c is provided between the extraction medium holding parts 54a and 54b. The extraction medium holding part 54a is so configured that the extraction medium flowing in from the liquid tank 55a is held in a region surrounded by the extraction medium holding parts 54a and the arm 110 of the subject, as shown in Fig. 4, and the extraction medium holding part 54b is so configured that the extraction medium flowing in from the liquid tank 55b is held in a region surrounded by the extraction medium holding parts 54b and the arm 110 of the subject.

The extraction medium holding parts 54a and 54b may be provided with detecting means (not shown) detecting that the extraction medium has flowed in the extraction medium from the liquid tanks 55a and 55b and confirming that the extraction medium has been held in the extraction medium holding parts 54a and 54b. This detecting means (not shown) can be configured to detect change in the resistance value by applying a minute voltage between the electrodes 53a and 53b.

As shown in Figs. 5 and 6, an opening 53c is formed in a part of the electrode 53a covering a side of the extraction medium holding part 54a in the arrow Z2 direction (see Fig. 6). This opening 53c is arranged at a substantially central part of an opening of the extraction medium holding part 54a on the arrow Z2 direction side. The opening 53c has a function of removing air inside the extraction medium holding part 54a when the extraction medium flows in the extraction medium holding part 54a from the liquid tank 55a and a function of allowing air to flow in the inside of the extraction medium holding part 54a when the extraction medium is transferred to the reaction cartridge 90 (see Fig. 4) described later.

An air vent 53e is formed in a part of the electrode 53b covering a side of the extraction medium holding part 54b in the arrow Z2 direction (see Fig. 6). This air vent 53e has a function of removing air inside the extraction medium holding part 54b when the extraction medium flows in the extraction medium holding part 54b from the liquid tank 55b.

The liquid tanks 55a and 55b store the extraction medium of pure water supplied to the extraction medium holding parts 54a and 54b, respectively. These liquid tanks 55a and 55b are connected to the extraction medium holding parts 54a and 54b respectively, and inner peripheral surfaces of the liquid tanks 55a and 55b are coated with water-repellent metal. Thus, the extraction medium of pure water can be smoothly supplied to the extraction medium holding parts 54a and 54b. According to the embodiment, the volumes of the liquid tanks 55a and 55b are a little larger than the volumes of the extraction medium holding parts 54a and 54b. Thus, the extraction medium can be derived from the opening 53c formed in the electrode 53a on an upper part of the extraction medium holding part 54a when the extraction medium flows in the extraction medium holding part 54a from the liquid tank 55a. The extraction medium derived from this opening 53c comes into contact with a transfer passage 92 described later, inserted in the vicinity of the opening 53c and is transferred to the detecting unit 60 (sensor member 94) by capillarity of the transfer passage 92.

The tape 52 is attached to cover the first surface (surface on the arrow Z2 direction side in Fig. 5) of each of the liquid tanks 55a and 55b. An air vent 52a is formed on the tape 52 covering the liquid tank 55a, and an air vent 52b is formed on the tape 52 covering the liquid tank 55b. These air vents 52a and 52b have a function of allowing air to flow in the liquid tanks 55a and 55b respectively when the extraction medium flows in the extraction medium holding parts 54a and 54b from the liquid tanks 55a and 55b respectively.

A negative voltage is applied to the electrode 53a, and a positive voltage is applied to the electrode 53b. Thus, a current flows in the extraction medium held in the extraction medium holding part 54a, the arm 110 of the subject and the extraction medium held in the extraction medium holding part 54b when voltages are applied to the electrodes 53a and 53b in a state where the extraction medium is held in the extraction medium holding parts 54a and 54b. At this time, the speed of extracting glucose from the arm 110 of the subject is increased.

The detecting unit 60 is configured to transfer the extraction medium to which glucose is extracted in the extraction unit 1 and has a function of detecting the glucose from the transferred extraction medium. The detecting unit 60 also has a function of acquiring the blood glucose level of the subject by analyzing a detection result of the glucose.

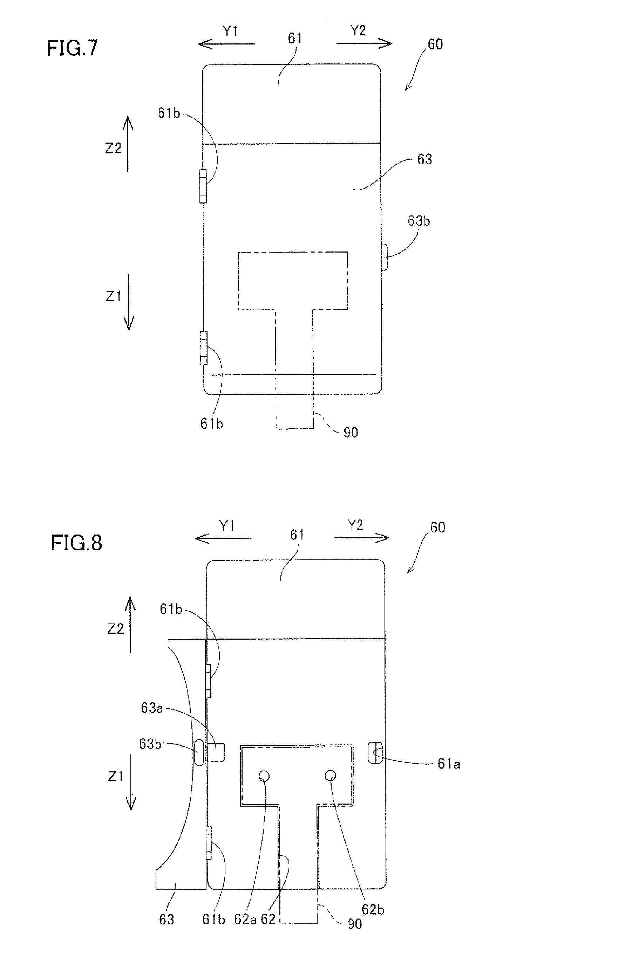

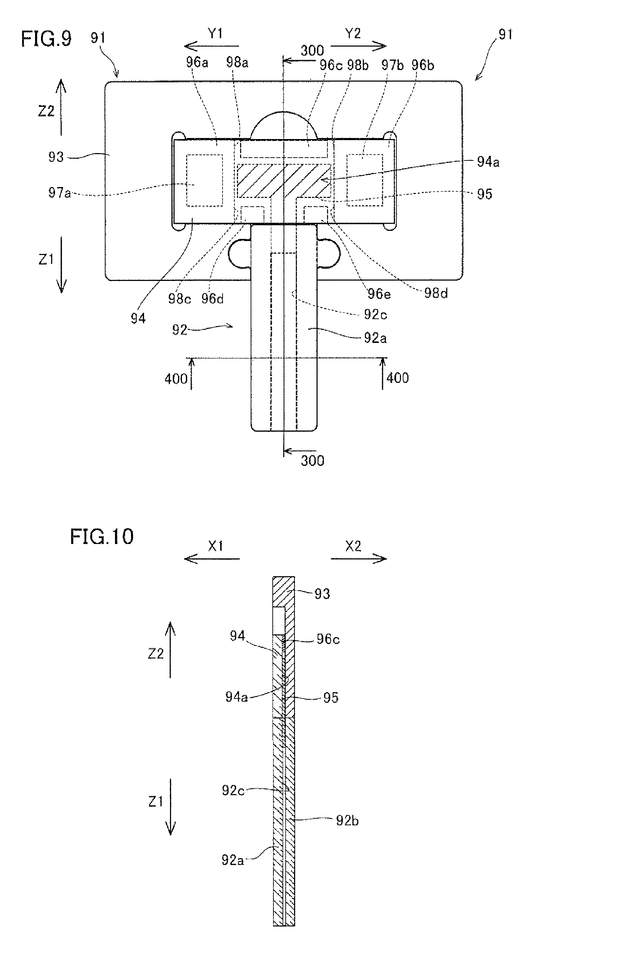

As shown in Figs. 1, 7 and 8, the detecting unit 60 is mainly configured by a detecting unit body 61, a cartridge holding part 62 (see Fig. 8) provided in the detecting unit body 61, holding the reaction cartridge 90, a door member 63 provided on a side of the detecting unit body 61 in the arrow X2 direction in Fig. 1, being openable/closable with respect to the cartridge holding part 62, and a display 64 and operation buttons 65 provided on a side of the detecting unit body 61 in the arrow X1 direction in Fig. 1. The reaction cartridge 90 arranged in the cartridge holding part 62 is mainly configured by a cartridge body 91 and the transfer passage 92 attached to the cartridge body 91 and is substantially T-shaped, as shown in Fig. 9.

The cartridge holding part 62 is substantially T-shaped to be capable of storing the reaction cartridge 90 and is configured to be capable of projecting the transfer passage 92 of the reaction cartridge 90 in an arrow Z1 direction, as shown in Fig. 8. In other words, the thickness of a part in which the transfer passage 92 of the cartridge holding part 62 is arranged, in the arrow Z1 direction is smaller than the length of the transfer passage 92 of the reaction cartridge 90. The cartridge holding part 62 is provided with two opening holes 62a and 62b. The opening hole 62a allows light irradiated from a light source 68 (see Fig. 4), described later, inside the detecting unit body 61 to pass therethrough and is provided for applying the light passing therethrough to a diffraction grating 97a (see Fig. 9), described later, of the reaction cartridge 90. The opening hole 62b is provided for incorporating light passing through to a diffraction grating 97b (see Fig. 9), described later, of the reaction cartridge 90 into the detecting unit body 61.

An engaging hole 61a is provided on a side of the cartridge holding part 62 of the detecting unit body 61 in the arrow Y2 direction, and a pair of hinge parts 61b are provided on a side of the cartridge holding part 62 of the detecting unit body 61 in the arrow Y1 direction. The door member 63 is attached to the pair of hinge parts 61b. Thus, the door member 63 is openable/closable with respect to the cartridge holding part 62. The door member 63 is provided with an engaging pawl 63a being capable of engaging with the engaging hole 61a of the detecting unit body 61 and a release button 63b being capable of releasing engagement of the engaging pawl 63a engaging with the engaging hole 61a and the engaging hole 61a. Thus, the door member 63 can be locked in a closed state with respect to the cartridge holding part 62 and can be easily unlocked.

The display 64 on the side of the detecting unit body 61 in the arrow X1 direction in Fig. 1 is provided for displaying the quantity of glucose and the blood glucose level calculated by a control part 66 (see Fig. 4) described later. The operation buttons 65 are provided for controlling the blood glucose meter 100 such as starting measurement on the basis of the subject's operation. The control part 66 for controlling the detecting unit 60 is connected to these display 64 and operation buttons 65, as shown in Fig. 4. The control part 66 is configured by a CPU, and a constant-voltage power supply 67 supplying power to the control part 66 and so on is connected to the control part 66. A light source 68 made of an LED and a detecting part 69 made of a photodiode are connected to the control part 66.

According to the embodiment, as shown in Figs. 9 and 10, the cartridge body 91 of the reaction cartridge 90 is configured by a base 93 made of resin, a sensor member 94 arranged on a side of the base 93 in the arrow X1 direction, and a mesh member 95 arranged to be sandwiched between the base 93 and the sensor member 94. The sensor member 94 is fixed to the base 93 by employing a plurality of two-sided tapes 96a, 96b, 96c, 96d and 96e.

The sensor member 94 has a measurement face 94a performing analysis of glucose in a central part (shaded area in Fig. 9) on a lower surface side (arrow X2 direction side) of a substrate made of glass in the arrow Y1 direction and the arrow Y2 direction. This measurement face 94a is coated with a mixed gel containing a chromogenic dye pigmenting due to reaction of glucose and prescribed enzymes, and subjected to treatment for drying the mixed gel. More specifically, the measurement face 94a of the sensor member 94 is coated with a mixed gel prepared by mixing glucose oxidase (GOD) which is oxidase serving as a catalyst for glucose, peroxidase (POD) which is oxidoreductase serving as a catalyst for hydrogen peroxide (H₂O₂) resulting from reaction of the glucose through catalytic action of GOD and a chromogenic dye pigmenting by reacting with O* (active oxygen) resulting from reaction of H₂O₂ through catalytic action of POD into a gel, and subjected to treatment for drying the mixed gel.

A pair of the diffraction gratings 97a and 97b are provided on the lower surface side (arrow X2 direction side) of the substrate of the sensor member 94 in the arrow Y1 direction and the arrow Y2 direction, respectively. The diffraction grating 97a is irradiated by the light source 68 (see Fig. 4) and has a function of diffracting the light (laser beam) passing through the opening hole 62a (see Fig. 8) on a side of the measurement face 94a, and the diffraction grating 97b has a function of diffracting the light passing through the measurement face 94a on a side of the opening hole 62b (see Fig. 8). The amount of this light is decreased by a pigmented color (blue color) when having passed through the measurement face 94a and in a case of pigmenting due to reaction of glucose. Thus, the amount of light detected by the detecting part 69 (see Fig. 4) changes in response to the quantity of glucose, and hence the blood glucose level can be calculated by the control part 66 (see Fig. 4) on the basis of the amount of change in the light detected by the detecting part 69.

The mesh member 95 extends in the arrow Y1 direction and the arrow Y2 direction between the diffraction grating 97a and the diffraction grating 97b and has a T shape diverging to projecting on a side (arrow Z1 direction side) of the transfer passage 92 from a middle part between the diffraction grating 97a and the diffraction grating 97b. A part of this mesh member 95 on the side of the transfer passage 92 and is configured to allow the extraction medium soaked up by the transfer passage 92 to smoothly move to a side of the measurement face 94a. The extraction medium is held substantially equally throughout the mesh member 95 and can be infiltrated into the overall measurement face 94a.

A clearance 98a having a prescribed width is formed between the two-sided tape 96a and the two-sided tape 96c, and a clearance 98b having a prescribed width is formed between the two-sided tape 96b and the two-sided tape 96c, as shown in Fig. 9. A clearance 98c having a prescribed width is formed between the two-sided tape 96a and the two-sided tape 96d, and a clearance 98d having a prescribed width is formed between the two-sided tape 96b and the two-sided tape 96e. The clearances 98a, 98b, 98c and 98d are provided for removing air existing between the base 93 and the sensor member 94 (in the mesh member 95). These clearances 98a, 98b, 98c and 98d are provided in the vicinities of upper and lower ends (ends in the arrow Z2 direction and the arrow Z1 direction) on a side of the mesh member 95 in the arrow Y1 direction and in the vicinities of upper and lower ends (ends in the arrow Z2 direction and the arrow Z1 direction) on a side of the mesh member 95 in the arrow Y2 direction, and hence air can be removed substantially equally from four directions of the upper and lower ends on the side of the mesh member 95 in the arrow Y1 direction and the upper and lower ends on the side of the mesh member 95 in the arrow Y2 direction. Thus, the extraction medium soaked up from the transfer passage 92 can be smoothly infiltrated into the overall mesh member 95.

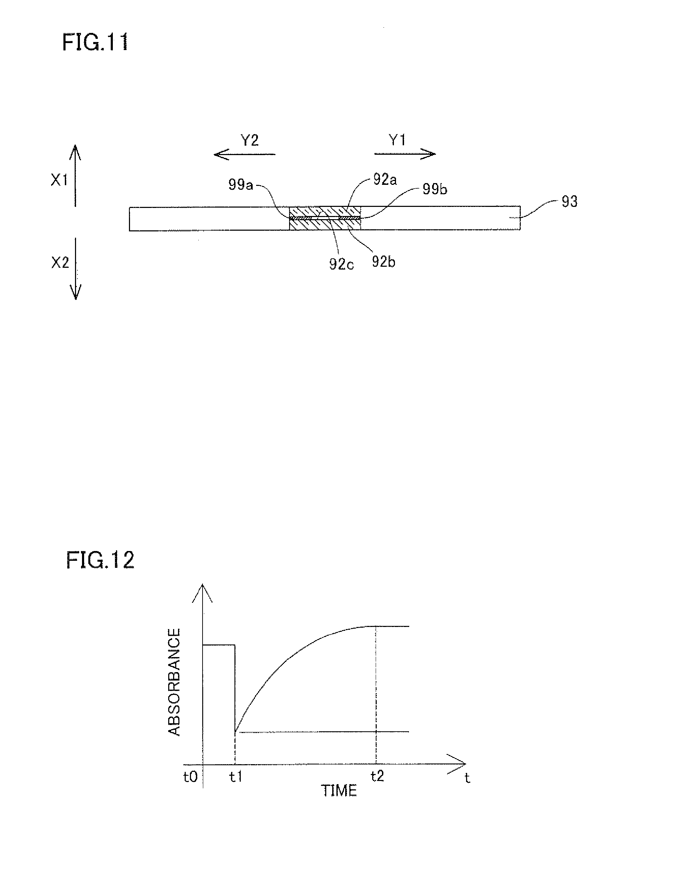

According to the embodiment, the transfer passage 92 is attached to the cartridge body 91 of the reaction cartridge 90. The transfer passage 92 is configured by two plates 92a and 92b made of resin and two-sided tapes 99a and 99b for attaching the two plates 92a and 92b to each other, as shown in Figs. 10 and 11. The transfer passage 92 is arranged to extend in the arrow Z1 direction from a part of the sensor member 94 on the arrow Z1 direction side, as shown in Fig. 10. A part 92c through which the extraction medium passes, of the transfer passage 92 is configured by a part surrounded by the two plates 92a and 92b made of resin and the two two-sided tapes 99a and 99b, as shown in Fig. 11. A thickness of the part 92c through which the extraction medium passes, of this transfer passage 92 in the arrow X1 direction and the arrow X2 direction is substantially the same as those of the two-sided tapes 99a and 99b. More specifically, these two-sided tapes 99a and 99b each have a thickness of about 90 µm. Hydrophilic polymer films are provided on surfaces of the plates 92a and 92b on a side of the part 92c through which the extraction medium passes. Thus, the extraction medium of the extraction medium holding part 54a coming into contact with a lower end (end in the arrow Z1 direction) of the transfer passage 92 can be transferred to the sensor member 94 (see Fig. 9) by capillarity.

According to the embodiment, the control part 66 has a function of monitoring that the extraction medium has been transferred from the extraction medium holding part 54a to the sensor member 94, as shown in Fig. 4. More specifically, as shown in Fig. 12, when the extraction medium is not infiltrated into the measurement face 94a (see Fig. 9) (in a case between time t0 and time t1), the light irradiated by the light source 68 (see Fig. 4) is reflected by air having a small refractive index, existing in a part surrounded by the base 93 and the measurement face 94a, and hence the amount of light entering the detecting part 69 is large. Thus, the light irradiated by the light source 68 is detected by the detecting part 69 (see Fig. 4) in a state where the amount of the light stays substantially constant. Thereafter, when the extraction medium is infiltrated into the measurement face 94a (in a case between time t1 and time t2), the air existing in the part surrounded by the base 93 and the measurement face 94a is replaced by the extraction medium having a relatively large refractive index, and hence the amount of reflected light is decreased and the amount of light entering the detecting part 69 is decreased. The control part 66 (see Fig. 4) can determine whether or not the extraction medium has been transferred to the sensor member 94 (see Fig .4) on the basis of this change in absorbance.

A use procedure of the blood glucose meter 100 according to the embodiment will be now described with reference to Figs. 2, 3, 4 and 13 to 18.

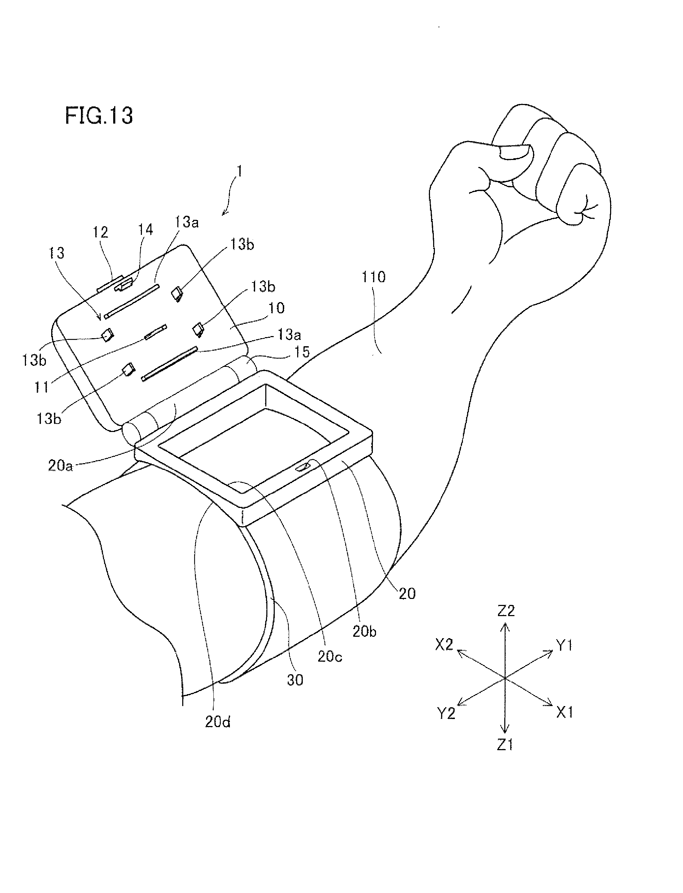

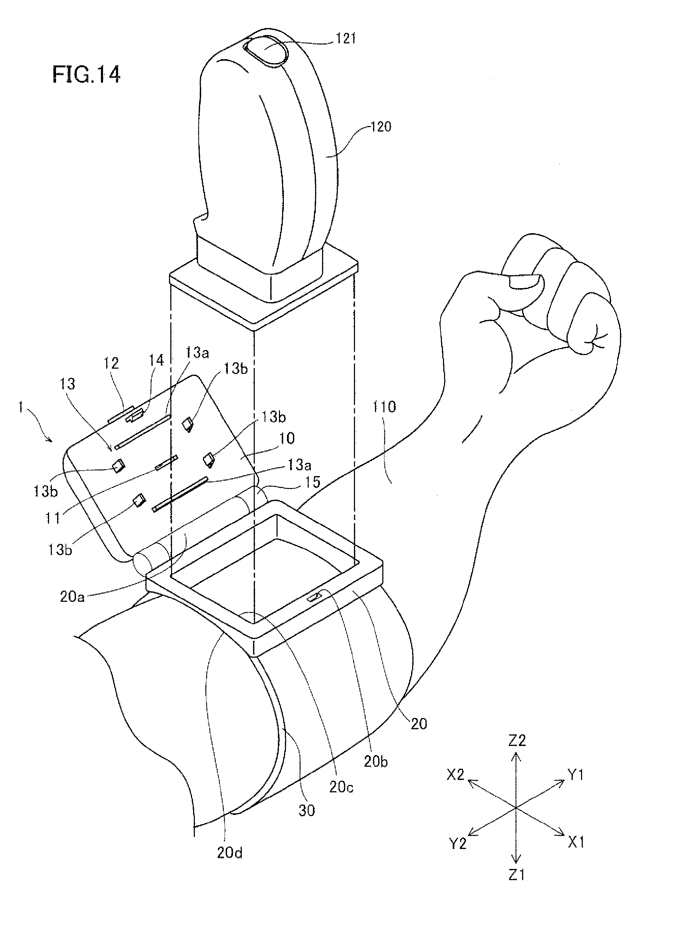

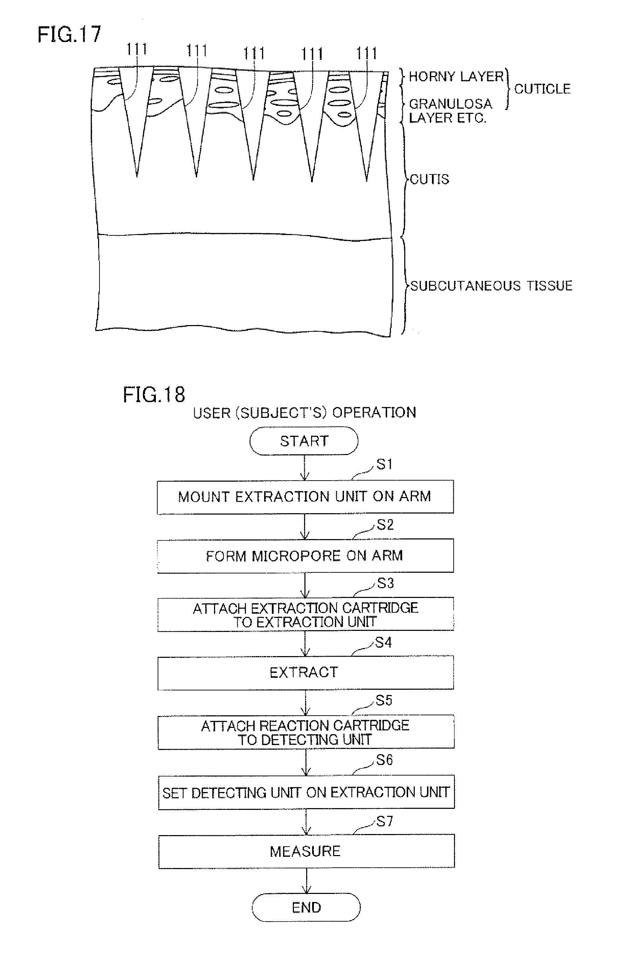

According to the embodiment, at a step S1 in Fig. 18, the holding member 20 is mounted on the arm 110 of the subject by wrapping the mounting belt 30 around the arm 110 of the subject, as shown in Fig. 13. At a step S2, micropores (extraction holes) 111 (see Fig. 17) are formed on the arm 110 of the subject by a micropore forming device 120 on the basis of the subject's operation, as shown in Fig. 14. More specifically, after fitting the micropore forming device 120 into the opening 20c, minute needles (not shown) protrude from the inside of the micropore forming device 120 by the subject pressing a button part 121. Thus, the micropores (extraction holes) 111 (see Fig. 17) are formed on the arm 110 of the subject. Thus, as shown in Fig. 17, the micropores (extraction holes) 111 passing through the cuticle and reaching the cutis without reaching the subcutaneous tissue are formed on the arm 110 of the subject, and hence body fluid can be extracted from the arm 110 of the subject through a plurality of these micropores (extraction holes) 111. Thus, pain felt by the subject can be eased when extracting glucose from the arm 110 of the subject with the blood glucose meter 100. After this, the micropore forming device 120 is detached from the extraction unit 1 (holding member 20).

Next, at a step S3, the extraction cartridge 50 is engaged with the four engaging parts 13b of the extraction unit 1 by the subject, as shown in Fig. 3. In other words, the extraction cartridge 50 is attached to the extraction cartridge attachment part 13.

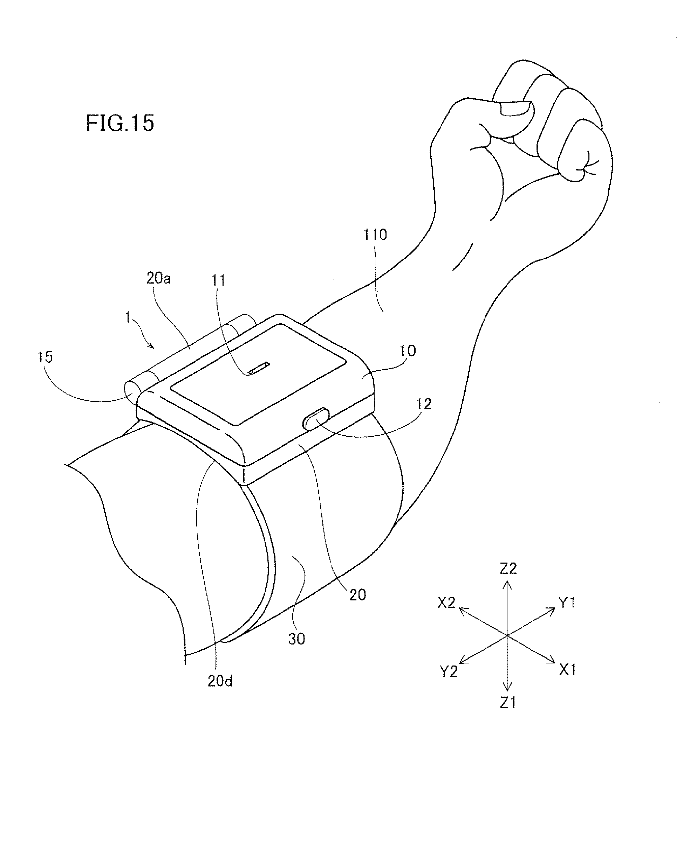

Thereafter, at a step S4, glucose is extracted from the arm 110 (micropores 111) of the subject to the extraction medium. More specifically, as shown in Fig. 15, the device body 10 is rotated in a direction B (see Fig. 2) to be rendered in a closed state. At this time, the engaging pawl 14 (see Fig. 3) of the device body 10 and the engaging hole 20b (see Fig. 3) of the holding member 20 engage with each other, and hence the rotation of the device body 10 in the direction A with respect to the holding member 20 is limited. At this time, the extraction medium holding parts 54a and 54b of the extraction cartridge 50 are in contact with the arm 110 of the subject, as shown in Fig. 4. The extraction medium is supplied from the liquid tanks 55a and 55b to the extraction medium holding parts 54a and 54b and is held in a part surrounded by the arm 110 of the subject and the extraction medium holding parts 54a and 54b. Then, glucose is extracted from the arm 110 (micropores 111) of the subject for a prescribed time in a state where the extraction medium is held in the part surrounded by the arm 110 of the subject and the extraction medium holding parts 54a and 54b. At this time, the constant-voltage power supply 16 is controlled to promote the extraction of the glucose by the control part 17 of the extraction unit 1. Control by this control part 17 will be described in detail later.

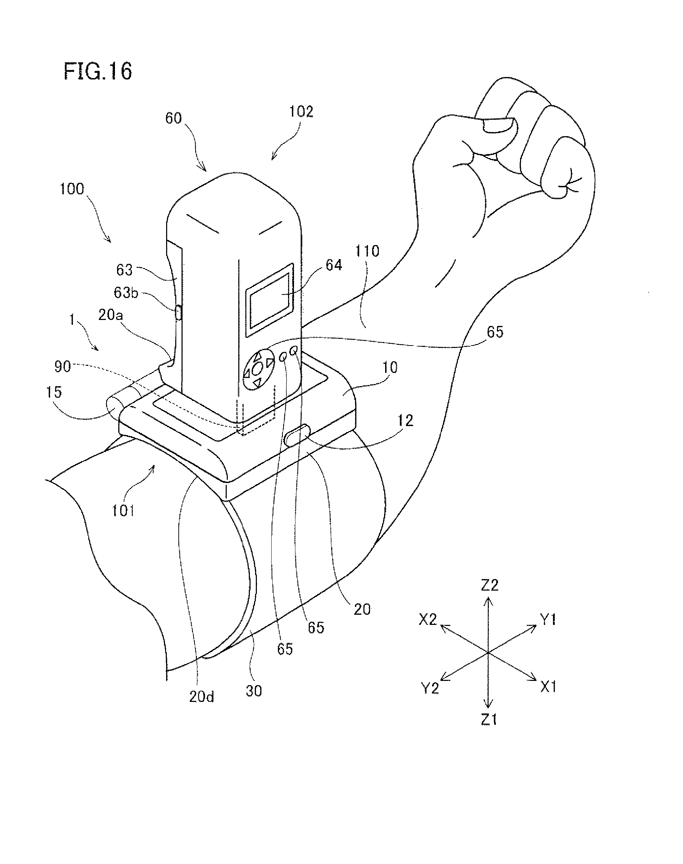

While the glucose is extracted from the arm 110 of the subject for the prescribed time at the aforementioned step S4 or after the glucose is extracted for the prescribed time, the reaction cartridge 90 is attached to the detecting unit 60 at a step S5. Thereafter, at a step S6, the detecting unit 60 to which the reaction cartridge 90 is attached is fitted into the detecting unit mounting hole 11 of the extraction unit 1 by the subject, as shown in Fig. 16.

At a step S7, the glucose is detected and the blood glucose level is measured by the control part 66 of the detecting unit 60. At this time, a measurement result of the measured blood glucose level is displayed on the display 64, and measurement by the blood glucose meter 100 is terminated. Operations of the control part 66 of the detecting unit 60 at this step S7 will be described in detail later.

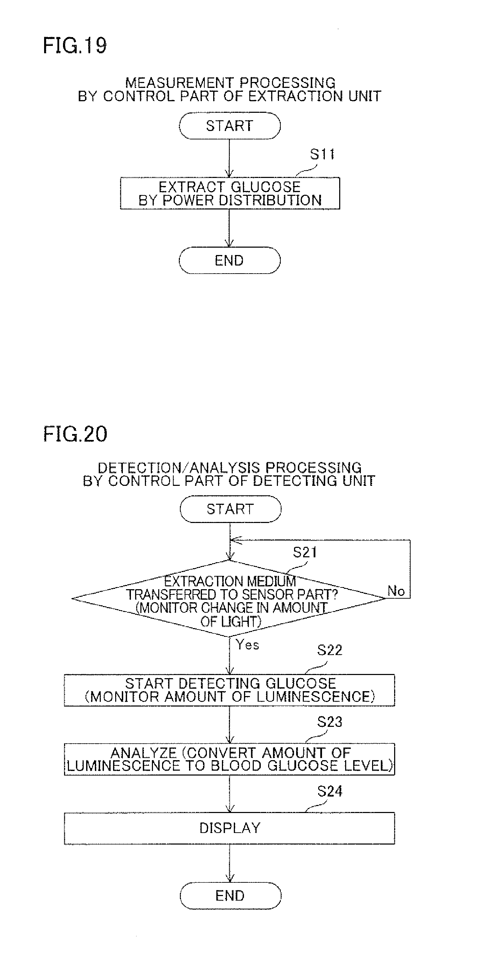

Fig. 19 is a flow chart for illustrating the operations of the control part when extracting glucose in the extraction unit of the blood glucose meter according to the embodiment shown in Fig. 1. Processing operations of the control part 17 when extracting the glucose in the extraction unit 1 of the blood glucose meter 100 according to the embodiment will be now described with reference to Figs. 4, 15 and 19.

According to the embodiment, at a step S11, the glucose is extracted from the arm 110 (micropores 111) of the subject for the prescribed time in a state where the extraction medium is held in the part surrounded by the arm 110 of the subject and the extraction medium holding parts 54a and 54b, as shown in Fig. 15. More specifically, the constant-voltage power supply 16 (see Fig. 4) applies a negative voltage to the electrode 53a (see Fig. 4) and a positive voltage to the electrode 53b (see Fig. 4) on the basis of the control by the control part 17 (see Fig. 4). Thus, the glucose is smoothly extracted from the arm 110 (micropores 111) of the subject toward the extraction medium in the extraction medium holding part 54a provided with the electrode 53a. After a lapse of the prescribed time, the constant-voltage power supply 16 stops applying the voltages to the electrodes 53a and 53b, and the processing operations of the control part 17 when extracting the glucose are terminated.

Processing operations of the control part 66 when detecting the glucose and measuring the blood glucose level in the detecting unit 60 of the blood glucose meter 100 according to the embodiment will be now described with reference to Figs. 4, 9, 12 and 20.

According to the embodiment, at a step S21, the control part 66 of the detecting unit 60 determines whether or not the extraction medium has been transferred to the sensor member 94, as shown in Fig. 20. More specifically, as shown in Fig. 12, the control part 66 determines whether or not the extraction medium has infiltrated into the measurement face 94a (see Fig. 9) of the sensor member 94 by passing through the transfer passage 92. The control part 66 determines that the extraction medium has been transferred to the sensor member 94 (measurement face 94a) (see Fig. 4) when large absorbance in a state where the extraction medium has not infiltrated into the measurement face 94a (in a state between time t0 and time t1) is changed to small absorbance in a state where the extraction medium has infiltrated into the measurement face 94a (in a state at time t1). When determining that the extraction medium has not been transferred to the sensor member 94 at the step S21, the control part 66 repeats the determination at the step S21. When determining that the extraction medium has been transferred to the sensor member 94 at the step S21, the control part 66 advances to a step S22.

Thereafter, at the step S22, the detection of the glucose starts. More specifically, the control part 66 continuously monitors absorbance when light of the light source 68 is absorbed by a chromogenic dye of the sensor member 94 from time t1 in Fig. 12. At a step S23, the control part 66 calculates the blood glucose level on the basis of absorbance when the absorbance becomes substantially saturated (at time t2). Then, at a step S24, the calculated blood glucose level is displayed on the display 64 by the control part 66, and the processing operations of the control part 66 when detecting the glucose and measuring the blood glucose level in the detecting unit 60 are terminated.

According to this embodiment, as hereinabove described, the reaction cartridge 90 including the sensor member 94 allowing glucose to cause a prescribed reaction and the transfer passage 92 for transferring the extraction medium held in the extraction medium holding parts 54a and 54b to the sensor member 94, configured to be detachable on the extraction medium holding parts 54a and 54b of the extraction unit 1 (extraction cartridge 50) is provided, whereby the reaction cartridge 90 can be attached to the extraction medium holding parts 54a and 54b of the extraction unit 1 and the glucose can be transferred from the extraction medium holding parts 54a and 54b to the sensor member 94 through the transfer passage 92 after extracting the glucose by mounting the extraction unit 1 in a state where the reaction cartridge 90 is detached on the arm 110 of the subject, and hence the subject does not have to mount a large-size and heavy apparatus at the time of extraction. Thus, a burden on the subject at the time of extraction can be reduced.

According to this embodiment, as hereinabove described, the transfer passage 92 is configured to transfer the extraction medium held in the extraction medium holding parts 54a and 54b to the sensor member 94 by capillarity, whereby the extraction medium held in the extraction medium holding parts 54a and 54b can be transferred to the sensor member 94 without a drive unit for transferring the extraction medium such as a pump, for example.

According to this embodiment, as hereinabove described, the transfer passage 92 is configured to come into contact with the extraction medium held in the extraction medium holding part 54a when the reaction cartridge 90 (detecting unit 60) is attached to the extraction medium holding part 54a of the extraction unit 1, whereby the extraction medium held in the extraction medium holding part 54a can be easily transferred.

According to this embodiment, as hereinabove described, the detecting unit 60 including the detecting part 69 and the cartridge holding part 62 detachably holding the reaction cartridge 90 is provided, whereby the detecting unit 60 enabling replacement of the reaction cartridge 90 can be obtained.

According to this embodiment, as hereinabove described, the detecting unit 60 is provided with the control part 66 analyzing a detection result, whereby the detecting unit 60 being capable of not only detecting glucose (extract) but also analyzing the detection result can be obtained.

According to this embodiment, as hereinabove described, the control part 66 monitoring that the extraction medium has been transferred from the extraction medium holding part 54a to the sensor member 94 is provided, whereby the control part 66 can be inhibited from performing an operation for analyzing the glucose in the extraction medium before the extraction medium is transferred to the sensor member 94.

According to this embodiment, as hereinabove described, the extraction unit 1 is provided with the holding member 20 and the mounting belt 30 detachably holding the extraction cartridge 50 and detachably mounted on the arm 110 of the subject, whereby the extraction unit 1 can be easily mounted on the arm 110 of the subject.

According to this embodiment, as hereinabove described, the extraction unit 1 is provided with the extraction cartridge 50 having the extraction medium holding parts 54a and 54b and the mounting belt 30 detachably holding the extraction cartridge 50 and detachably mounted on the arm 110 (extract extraction position) of the subject, whereby the extraction cartridge 50 can be exchanged every measurement, and no cleaning of the extraction unit 1 contaminated by the extract is required.

According to this embodiment, as hereinabove described, the extraction cartridge 50 is provided with the liquid tanks 55a and 55b storing the extraction medium held in the extraction medium holding parts 54a and 54b, connected to the extraction medium holding parts 54a and 54b, whereby the extraction medium can be directly supplied from the liquid tanks 55a and 55b provided in the extraction cartridge 50 to the extraction medium holding parts 54a and 54b, and hence the extraction medium can be inhibited from being mixed with impurities as compared with a case where the extraction medium is supplied externally.

According to this embodiment, as hereinabove described, the extraction unit 1 is provided with the terminals 13c and 13d promoting the extraction of the glucose and the extraction cartridge 50 is provided with the electrodes 53a and 53b being in contact with the terminals 13c and 13d, whereby the glucose can be extracted to the extraction medium supplied to the extraction medium holding parts 54a and 54b in less time.

According to this embodiment, as hereinabove described, the control part 66 analyzing the glucose (extract) is configured to analyze a detection result of the detecting part 69 and acquire the blood glucose level, whereby the blood glucose meter 100 being capable of acquiring the blood glucose level based on the detection result of the extracted glucose can be obtained.

According to this embodiment, as hereinabove described, the transfer passage 92 configured to transfer the extraction medium to the sensor member 94 by capillarity is provided, whereby the extraction medium held in the extraction medium holding part 54a can be transferred to the sensor member 94 without a drive unit for transferring the extraction medium such as a pump, for example.

According to this embodiment, as hereinabove described, the extraction medium holding parts 54a and 54b holding the extraction medium and the liquid tanks 55a and 55b storing the extraction medium held in the extraction medium holding parts 54a and 54b, connected to the extraction medium holding parts 54a and 54b are provided, whereby the extraction medium can be directly supplied from the liquid tanks 55a and 55b to the extraction medium holding parts 54a and 54b, and hence the extraction medium can be inhibited from being mixed with impurities as compared with a case where the extraction medium is supplied externally. The extraction medium holding parts 54a and 54b are provided with the air vents 52a and 52b for moving the extraction medium from the liquid tanks 55a and 55b to the extraction medium holding parts 54a and 54b, whereby the extraction medium can be smoothly moved to the extraction medium holding parts 54a and 54b following discharge of air existing in the extraction medium holding parts 54a and 54b from the air vents 52a and 52b.

The embodiment disclosed this time must be considered as illustrative in all points and not restrictive. The range of the present invention is shown not by the above description of the embodiment but by the scope of claims for patent, and all modifications within the meaning and range equivalent to the scope of claims for patent are further included.

For example, while the blood glucose meter has been described as an example of a biological component analyzer in the aforementioned embodiment, the present invention is not restricted to this, but the present invention can also be applied to another biological component analyzer.

While the example of forming the micropores (extraction holes) on the subject's arm by the micropore forming device on the basis of the subject's operation and promoting the extraction of the glucose by applying voltages to the electrodes has been shown in the aforementioned embodiment, the present invention is not restricted to this, but either the aforementioned step of forming the micropores (extraction holes) on the subject's arm or the aforementioned step of promoting the extraction of the glucose by applying voltages to the electrodes may be omitted.

While the example of providing both of the detecting part and the control part in the detecting unit has been shown in the aforementioned embodiment, the present invention is not restricted to this, but the detecting part and the control part may be configured by separate devices. In this case, the detecting unit is preferably provided with a transmitter being capable of transmitting a detection result to the detecting unit via radio waves, and the analyzer is preferably provided with a receiver receiving the detection result.

While the example of the structure allowing the extraction medium to flow in the extraction medium holding part from the liquid tank has been shown in the aforementioned embodiment, the present invention is not restricted to this, but the extraction medium may be previously stored in the extraction medium holding part.

While the example of attaching the reaction cartridge to the detecting unit by arranging the reaction cartridge in the cartridge holding part and covering the cartridge holding part by the door member has been shown in the aforementioned embodiment, the present invention is not restricted to this, but the reaction cartridge may be attached to the detecting unit by providing an engaging part being capable of engaging with the detecting unit and the reaction cartridge and enabling the reaction cartridge to be directly attached to the detecting unit.

While the example of arranging (inserting) the transfer passage in the vicinity of the opening of the electrode provided on the extraction medium holding part and transferring the extraction medium flowing out from the opening to the sensor member has been shown in the aforementioned embodiment, the present invention is not restricted to this, but the extraction medium in the extraction medium holding part may be directly transferred by inserting the transfer passage in the opening not in the vicinity of the opening of the electrode provided on the extraction medium holding part.

## Claims

1. A method of analyzing a biological component comprising steps of:
arranging an extraction medium holding device comprising an extraction medium holding part configured to be capable of holding an extraction medium for holding an extract extracted from a subject on the skin of the subject;
extracting said extract from the subject to said extraction medium held in said extraction medium holding part;
attaching a reaction cartridge comprising a reaction part allowing said extract to cause a prescribed reaction, configured to be detachable on said extraction medium holding part of said extraction medium holding device and to be capable of transferring said extraction medium held in said extraction medium holding part to said reaction part to said extraction medium holding part of said extraction medium holding device arranged on said skin;
transferring said extraction medium held in said extraction medium holding part to said reaction part;
detecting said extract from said prescribed reaction caused in said reaction part; and
analyzing a result of detecting said extract.

2. The method of analyzing a biological component according to claim 1, wherein
said step of transferring said extraction medium to said reaction part includes a step of transferring said extraction medium through a transfer passage provided in said reaction cartridge for transferring said extraction medium to said reaction part.

3. The method of analyzing a biological component according to claim 2, wherein
said step of transferring said extraction medium to said reaction part includes a step of transferring said extraction medium held in said extraction medium holding part to said reaction part through said transfer passage by capillarity.

4. A biological component analyzer, comprising:
an extraction medium holding device having an extraction medium holding part configured to be capable of holding an extraction medium for holding an extract extracted from a subject;
a reaction cartridge including a reaction part allowing said extract to cause a prescribed reaction and a transfer passage for transferring said extraction medium held in said extraction medium holding part to said reaction part, configured to be detachable on said extraction medium holding part of said extraction medium holding device;
a detecting part detecting said extract from said prescribed reaction caused in said reaction part; and
an analysis part analyzing a result of detecting said extract.

5. The biological component analyzer according to claim 4, wherein
said transfer passage is configured to transfer said extraction medium held in said extraction medium holding part to said reaction part by capillarity.

6. The biological component analyzer according to claim 4 or 5, wherein
said transfer passage is configured to come into contact with said extraction medium held in said extraction medium holding part when said reaction cartridge is attached to said extraction medium holding part of said extraction medium holding device.

7. The biological component analyzer according to any one of claims 4 to 6, provided with a detecting device including said detecting part and a cartridge holding part detachably holding said reaction cartridge.

8. The biological component analyzer according to claim 7, wherein
said detecting device includes said analysis part.

9. The biological component analyzer according to any one of claims 4 to 8, further comprising transfer monitoring means monitoring that said extraction medium has been transferred from said extraction medium holding part to said reaction part.

10. The biological component analyzer according to any one of claims 4 to 9, wherein
said extraction medium holding device includes an extraction cartridge having said extraction medium holding part and a mounting part detachably holding said extraction cartridge, detachably mounted on an extract extraction position of the subject.

11. The biological component analyzer according to claim 10, wherein
said extraction cartridge includes a liquid tank storing said extraction medium held in said extraction medium holding part, connected to said extraction medium holding part.

12. The biological component analyzer according to any one of claims 4 to 11, wherein
said extraction medium holding device further comprises an extraction promoting part promoting extraction of said extract.

13. The biological component analyzer according to any one of claims 4 to 12, wherein
said extract is glucose, and
said analysis part analyzes a detection result of said detecting part and acquires a blood glucose level.

14. A reaction cartridge, being employed in a biological component analyzer analyzing an extract extracted from a subject,
comprising:
a reaction part allowing the extract to cause a prescribed reaction, and
a transfer passage configured to transfer an extraction medium to said reaction part by capillarity.

15. An extraction cartridge employed in a biological component analyzer analyzing an extract extracted from a subject,
comprising:
an extraction medium holding part holding an extraction medium;
a liquid tank storing said extraction medium held in said extraction medium holding part, connected to said extraction medium holding part; and
an air vent provided in said extraction medium holding part for moving said extraction medium from said liquid tank to said extraction medium holding part.

## Amended claims

### Amended claims under Art. 19.1 PCT

**1.** A method of analyzing a biological component comprising steps of:
arranging an extraction medium holding device comprising an extraction medium holding part configured to be capable of holding an extraction medium for holding an extract extracted from a subject on the skin of the subject;
extracting said extract from the subject to said extraction medium held in said extraction medium holding part;
attaching a reaction cartridge comprising a reaction part allowing said extract to cause a prescribed reaction, configured to be detachable on said extraction medium holding part of said extraction medium holding device and to be capable of transferring said extraction medium held in said extraction medium holding part to said reaction part to said extraction medium holding part of said extraction medium holding device arranged on said skin;
transferring said extraction medium held in said extraction medium holding part to said reaction part;
detecting said extract from said prescribed reaction caused in said reaction part; and
analyzing a result of detecting said extract.

**2.** The method of analyzing a biological component according to claim 1, wherein
said step of transferring said extraction medium to said reaction part includes a step of transferring said extraction medium through a transfer passage provided in said reaction cartridge for transferring said extraction medium to said reaction part.

**3.** The method of analyzing a biological component according to claim 2, wherein
said step of transferring said extraction medium to said reaction part includes a step of transferring said extraction medium held in said extraction medium holding part to said reaction part through said transfer passage by capillarity.

**4.** A biological component analyzer, comprising:
an extraction medium holding device having an extraction medium holding part configured to be capable of holding an extraction medium for holding an extract extracted from a subject;
a reaction cartridge including a reaction part allowing said extract to cause a prescribed reaction and a transfer passage for transferring said extraction medium held in said extraction medium holding part to said reaction part, configured to be detachable on said extraction medium holding part of said extraction medium holding device;
a detecting part detecting said extract from said prescribed reaction caused in said reaction part; and
an analysis part analyzing a result of detecting said extract.

**5.** The biological component analyzer according to claim 4, wherein
said transfer passage is configured to transfer said extraction medium held in said extraction medium holding part to said reaction part by capillarity.

**6.** The biological component analyzer according to claim 4 or 5, wherein
said transfer passage is configured to come into contact with said extraction medium held in said extraction medium holding part when said reaction cartridge is attached to said extraction medium holding part of said extraction medium holding device.

**7.** The biological component analyzer according to any one of claims 4 to 6, provided with a detecting device including said detecting part and a cartridge holding part detachably holding said reaction cartridge.

**8.** The biological component analyzer according to claim 7, wherein
said detecting device includes said analysis part.

**9.** The biological component analyzer according to any one of claims 4 to 8, further comprising transfer monitoring means monitoring that said extraction medium has been transferred from said extraction medium holding part to said reaction part.

**10.** The biological component analyzer according to any one of claims 4 to 9, wherein
said extraction medium holding device includes an extraction cartridge having said extraction medium holding part and a mounting part detachably holding said extraction cartridge, detachably mounted on an extract extraction position of the subject.

**11.** The biological component analyzer according to claim 10, wherein
said extraction cartridge includes a liquid tank storing said extraction medium held in said extraction medium holding part, connected to said extraction medium holding part.

**12.** The biological component analyzer according to any one of claims 4 to 11, wherein
said extraction medium holding device further comprises an extraction promoting part promoting extraction of said extract.

**13.** The biological component analyzer according to any one of claims 4 to 12, wherein
said extract is glucose, and
said analysis part analyzes a detection result of said detecting part and acquires a blood glucose level.

**14.** (amended) A reaction cartridge, detachably attached to a biological component analyzer including an extraction medium holding device comprising an extraction medium holding part configured to be capable of holding an extraction medium for holding an extract extracted from a subject,
comprising:
a reaction part allowing the extract to cause a prescribed reaction, and
a transfer passage so configured that one end thereof comes into contact with said extraction medium held in said extraction medium holding part and said extraction medium is transferred to said reaction part by capillarity, when attaching said reaction cartridge to said extraction medium holding device.

**15.** An extraction cartridge employed in a biological component analyzer analyzing an extract extracted from a subject,
comprising:
an extraction medium holding part holding an extraction medium;
a liquid tank storing said extraction medium held in said extraction medium holding part, connected to said extraction medium holding part; and
an air vent provided in said extraction medium holding part for moving said extraction medium from said liquid tank to said extraction medium holding part.
